# EUROPÄISCHE PATENTSCHRIFT

(11) **EP 2 004 599 B1**
(45) Veröffentlichungstag und Bekanntmachung des Hinweises auf die Patenterteilung: **09.11.2011**
(21) Anmeldenummer: 07727480.1
(22) Anmeldetag: 29.03.2007
(51) Int. Cl.: C07D 201/18, C07D 211/76, C07D 223/10, C07D 207/26

(54) **Verfahren zur Herstellung eines Nn-Alkyl-Lactams mit verbesserter Farbqualität**
Process for the preparation of an n-alkyl lactam with improved colour quality
Procédé de fabrication d'un n-alkyl-lactame ayant une qualité de couleur améliorée

(30) Priorität: 06.04.2006 EP 06112323
(43) Veröffentlichungstag der Anmeldung: 24.12.2008
(73) Patentinhaber: BASF SE, 67056 Ludwigshafen (DE)
(72) Erfinder: WABNITZ, Tobias, 68169 Mannheim (DE); PINKOS, Rolf, 67098 Bad Dürkheim (DE); OTT, Karl, 68723 Plankstadt (DE)
(86) Internationale Anmeldenummer: PCT/EP2007/053008
(87) Internationale Veröffentlichungsnummer: WO 2007/115943

(56) Entgegenhaltungen:
- US-A- 4 168 226
- DATABASE WPI DERWENT PUBLICATIONS LTD., LONDON, GB; XP002456960 Database accession no. 1986-262694[40] & JP 61 191674 A (MITSUBISHI CHEM IND LTD) 26. August 1986 (1986-08-26)
- DATABASE CAPLUS CHEMICAL ABSTRACTS SERVICE, COLUMBUS, OHIO, US; XP002457008 gefunden im STN Database accession no. 1995:297680 & JP 06 279401 A (MITSUBISHI CHEMICAL INDUSTRIES CO LTD) 4. Oktober 1994 (1994-10-04) in der Anmeldung erwähnt

## Beschreibung

Die vorliegende Erfindung betrifft ein Verfahren zur Herstellung eines N-Alkyl-lactams mit verbesserter Farbqualität und Mischungen enthaltend mindestens 99,0 Gew.-% eines N-Alkyl-lactams und im Bereich von 100 bis 5000 Gew.-ppm eines C₁₋₁₀-Alkohols oder eines Acetals, Aminals oder eines Orthoesters, das/der im Bereich von 100 bis 5000 Gew.-ppm eines C₁₋₁₀-Alkohols freisetzt.

N-Alkyl-lactame sind wichtige Produkte in der chemischen Industrie. Am weitesten verbreitet sind unter ihnen N-Alkyl-pyrrolidone (N-Alkyl-lactame mit fünfgliedrigen Ring).

N-Alkyl-pyrrolidone sind z.B. organische Lösungsmittel, die in einer Vielzahl von Anwendungen zum Einsatz kommen.
N-Alkyl-pyrrolidone sind thermisch stabile, chemisch weitgehend inerte, farblose, niedrigviskose und aprotische Lösungsmittel mit breiter Anwendbarkeit. So sind N-Methyl-pyrrolidon (NMP) und N-Ethyl-pyrrolidon (NEP) sowie die höheren Homologen als Lösungsmittel, Verdünnungsmittel, Extraktionsmittel, Reinigungsmittel, Entfettungsmittel, Adsorptionsmittel und/oder Dispergierungsmittel einsetzbar.

NMP findet Anwendung in der Extraktion von reinen Kohlenwasserstoffen in der petrochemischen Verarbeitung, in der Reinigung und Abtrennung von Gasen wie Acetylen 1,3-Butadien oder Isopren, in der Aromatenextraktion, beispielsweise im Distapex-Verfahren der LURGI GmbH, in der Sauergaswäsche und in der Schmierölextraktion. Außerdem kann NMP als Lösungsmittel für Polymer-Dispersionen, beispielsweise für Polyurethan-Dispersionen, verwendet werden.

NMP ist auch ein gutes Lösungsmittel für viele Kunststoffe wie Polyvinylchlorid (PVC), Polyurethane (PU), Acrylate oder Butadien-Acrylnitril-Copolymere und wird bei deren Verarbeitung eingesetzt.

NMP wird auch als Reinigungsmittel bei der Entfernung von Farb- und Lackresten eingesetzt sowie als Abbeizer und als Reinigungsmittel für Metall-, Keramik-, Glas- und Kunststoffoberflächen.

Ebenso ist NMP Lösungsmittel oder Cosolvens für die Formulierung von Wirkstoffen im Pflanzenschutz.

NEP und andere N-Alkyl-pyrrolidone können NMP in vielen Anwendungen ersetzen und zeigen darüber hinaus in vielen Fällen zusätzlich vorteilhafte Eigenschaften (WO-A-2005/090447, BASF AG).

Andere verbreitete N-Alkyl-lactame sind N-Alkyl-piperidone und N-Alkyl-caprolactame. N-Alkyl-caprolactame, insbesondere N-Methyl-caprolactam, können als Selektivlösungsmittel zur Gasentsäuerung eingesetzt werden, wie in Chem. Techn. 29 (1977), Seiten 445-448 (Wehner et al., VEB Leuna) beschrieben. Auch bei der Extraktion von Kohlenwasserstoffen finden N-Alkyl-caprolactame aufgrund der hohen erreichbaren Selektivitäten Anwendung, wie in Chem. Techn. 27 (1975), Seiten 401-405 (Wehner et al., VEB Leuna) beschrieben. Vergl auch WO-A-05/092953 (BASF AG). N-Alkyl-piperidone, wie beispielsweise N-Methyl-piperidon, können in diesen Anwendungen ebenfalls zum Einsatz kommen.

Die Herstellung von N-Alkyl-lactamen ist bekannt. N-Alkyl-pyrrolidone können beispielsweise durch Umsetzung von gamma-Butyrolacton (y-BL) mit Monoalkylaminen erfolgen unter Freisetzung von einem Äquivalent Wasser, z. B. analog Ullmann's Encyclopedia of Industrial Chemistry, Band A22, 5. Aufl., S. 459 (1993) oder analog DE-A-19 626 123 (BASF AG). Ebenso können N-Alkyl-pyrrolidone aus Maleinsäureanhydrid oder anderen Dicarbonsäurederivaten und Monoethylaminen in Gegenwart von Wasserstoff und einem Hydrierkatalysator hergestellt werden, z. B. gemäß EP-A-745 598 (Bayer AG) oder WO-A-02/102773 (BASF AG).

Andere N-Alkyl-lactame wie N-Alkyl-piperidone und N-Alkyl-caprolactame können ebenfalls aus den entsprechenden Lactonen durch Umsatz mit Monoalkylaminen hergestellt werden, wie beispielsweise durch Yakugaku Zasshi 71 (1951), 1341 (Susagawa et al.) beschrieben. Außerdem können diese Lactame auch durch Umsetzung von O-xynitrilen mit Monomethylaminen erhalten werden, wie in DE-A-11 92 208 (BASF AG) offenbart, oder auch elegant durch Umsatz von Lactamen mit Monoalkoholen oder Dialkylethern an sauren Katalysatoren wie Al₂O₃, wie in Chem. Techn. 33 (1981), 193-196 (Wehner et al., VEB Leuna) oder RO 137218 (Centrul de Cercetari pentru Fibre Chimice) beschrieben, oder auch mit anderen Alkylierungsmitteln wie Dialkylsulfaten oder Alkylhalogeniden unter basischen Bedingungen, wie beispielsweise in J. Org. Chem. 29 (1964), Seiten 2748-2750 (Moriarty) beschrieben.

Reinigungsverfahren für N-Alkyl-lactame sind bekannt. Die Reinigung von N-Alkyl-pyrrolidonen kann beispielsweise durch fraktionierte Destillation (auch mehrfach, wie in JP 06 228 088 (Mitsubishi Kasei Corp.) beschrieben) oder durch Extraktion erfolgen. Andere oder zusätzliche Reinigungsschritte können die Behandlung mit lonentauschern, wie in beispielsweise in EP-A-1 038 867 (BASF AG) beschrieben, oder mit festen Adsorbentien wie Aluminiumoxid analog WO-A-2005/092851 (Lyondell L.P.) sein. N-Alkyl-pyrrolidone können außerdem in Gegenwart von Säuren wie Toluolsulfonsäure (beschrieben z. B. JP 11 071 346 (Tonen Corp.)) oder Phosphorsäure (beschrieben z. B. in JP 2028148 (Ouchi Shinko Chem.)) während der Destillation gereinigt werden. Andere vorteilhafte Zusätze während der Herstellung und/oder Destillation können Alkali-, Erdalkali- oder Ammoniumborhydride wie beispielsweise in

US 4,885,371 (GAF Chemicals Corp.) offenbart, Oxidationsmittel wie Kalimpermanganat, Natriumperborat oder Kaliumdichromat, wie in JP 72 22 225 (Teijin Ltd.) beschrieben oder Natriumhydroxid wie in US 2,964,535 (Monsanto Chemicals) beschrieben, sein.

Außerdem lehrt JP-A-2001 089 446 (Mitsubishi Chem. Corp.), dass sauberes NMP mit geringer Farbe erhalten werden kann, wenn während der Destillation die Mengen an Wasserstoff und Sauerstoff Grenzwerte von 0,01 mol-% und 0,002 mol-% bezogen auf den Pyrrolidongehalt nicht überschreiten. Nach JP 62 79 401 (Mitsubishi Kasei Corp.) kann farbloses N-Methyl-pyrrolidon auch durch thermische Behandlung ("Tempern" bei 150-250°C) und anschließende Destillation erhalten werden.
Andere N-Alkyl-lactame wie N-Alkyl-piperidone und N-Alkyl-caprolactame können auf analogen Wegen gereinigt werden.

Für viele Anwendungen von N-Alkyl-lactamen, beispielsweise dem Einsatz von N-Alkyl-pyrrolidonen als Lösungsmittel bei der Herstellung von Farben und Lacken oder Klebstoffen oder in der Produktion von Kunststoffen, ist es wichtig, dass diese Lösungsmittel in möglichst farbloser, d.h. nicht vergilbter Form eingesetzt werden.

N-Alkyl-pyrrolidone neigen bei Lagerung zur Vergilbung und kommen dann für solche Anwendungen nicht mehr in Frage, da ein Teil der Verfärbung in den Produkten wie Lacken oder Kunststoffen erhalten bleibt und dort unerwünscht ist.

Methoden zur Entfernung von Vergilbungen durch Reinigung oder sonstige Behändlung von N-Alkyl-pyrrolidonen und zur Vermeidung der Vergilbung während der Lagerung sind daher wichtig.

Es ist bekannt, dass Reinigungsverfahren wie fraktionierende Destillationen, Mehrfachdestillation, Destillationen in Gegenwart von Zusätzen wie Säuren (z. B. p-Toluolsulfonsäure), Basen (z. B. Natriumhydroxid), Reduktionsmitteln (z. B. Natriumborhydrid) und Oxidationsmitteln (z. B. Kaliumpermanganat) zu einer Verbesserung, d.h. zu einer Aufhellung, der Farbe von N-Alkyl-pyrrolidonen führen können.

Es ist außerdem bekannt, dass die Entfernung von Verunreinigungen durch Behandlung mit Aluminiumoxid oder makroporösen lonenaustauscherharzen zu einer Verbesserung von Produkteigenschaften einschließlich der Farbe führen kann.

Außerdem ist bereits beschrieben, dass Zusätze wie z. B. Natriumborhydrid während der Synthese von N-Alkyl-pyrrolidonen zu verbesserten Farbeigenschaften führen können.

Während für Reinigungs- und Herstellungsverfahren zahlreiche dieser Methoden zur Verbesserung der Farbeigenschaften beschrieben sind, gibt es nur sehr wenige technische Lösungen zur Stabilisierung des Produkts während der Lagerung.

In JP-A-2003 081 885 (Mitsubishi Chem. Corp.) wird beschrieben, dass die Sättigung und Überlagerung von N-Methyl-pyrrolidon mit Stickstoff, so dass nur sehr geringe Mengen Sauerstoff zurückbleiben, die Lagerstabilität stark verbessert und die Geschwindigkeit der oxidativen Degradation deutlich herabsetzt. Die thermische Stabilität gegenüber Zersetzung bei hohen Temperaturen (oberhalb 250 °C) von N-Methyl-pyrrolidon kann nach US 4,168,226 (Exxon Research & Engineering Co.) auch dadurch verbessert werden, dass bis zu 0,5 Gew.-% Wasser zugesetzt werden. In diesen Fällen verläuft die Zersetzung von NMP stark verlangsamt ab.

Der vorliegenden Erfindung lag die Aufgabe zugrunde, eine Methode zur Vermeidung und/oder Verlangsamung der Vergilbung von N-Alkyl-lactamen während Verarbeitung und Lagerung aufzufinden. Es sollte ein verbessertes, wirtschaftliches, einfach durchzuführendes Verfahren zur Herstellung eines N-Alkyl-lactams mit verbesserter Farbqualität, d.h. Verminderung der Verfärbung und/oder Verbesserung der Farbstabilität, insbesondere bei Lagerung, aufgefunden werden.

In JP 61191674 A offenbart eine Zusammensetzung aus 99,9 Gew.-% N-Methyl-2-pyrrolidon, 0,002 Gew.-% (20 Gew.-ppm) Ethylenglykol und 0,003 Gew.-% (30 Gew.-% Ethylbenzol. Der Einsatz von C₁₋₁₀-Alkoholen in einem Bereich von 100 bis 100000 ppm innerhalb einer solchen Mischung um eine besserte Farbqualität bei thermischen Stabilisierung zu erhalten wird in JP 61191674 A nicht offenbart.

Demgemäß wurde ein Verfahren zur Herstellung eines N-Alkyl-lactams gefunden, welches dadurch gekennzeichnet ist, dass man dem N-Alkyl-lactam im Bereich von 0,01 bis 10 Gew.-% eines C₁₋₁₀-Alkohols oder eine Verbindung, die im Bereich von 0,01 bis 10 Gew.-% eines C₁₋₁₀-Alkohols freisetzt, zusetzt.

Weiterhin gefunden wurden Mischungen enthaltend mindestens 99,0 Gew.-% eines N-Alkyl-lactams und im Bereich von 100 bis 5000 Gew.-ppm eines C₁₋₁₀-Alkohols oder eines Acetals, Aminals oder eines Orthoesters, das/der im Bereich von 100 bis 5000 Gew.-ppm eines C₁₋₁₀-Alkohols freisetzt.

Das Verfahren ist bevorzugt **dadurch gekennzeichnet, dass** man dem N-Alkyl-lactam im Bereich von 0,02 bis 2 Gew.-%, insbesondere 0,03 bis 1 Gew.-%, besonders 0,03 bis 0,5 Gew.-%, weiter besonders 0,03 bis 0,2 Gew.-%, weiter besonders 0,03 bis 0,1 Gew.-%, eines C₁₋₁₀-Alkohols oder eine Verbindung, die im Bereich von 0,02 bis 2 Gew.-%, insbesondere 0,03 bis 1 Gew.-%, besonders 0,03 bis 0,5 Gew.-%, weiter besonders 0,03 bis 0,2 Gew.-%, weiter besonders 0,03 bis 0,1 Gew.-%, eines C₁₋₁₀-Alkohols freisetzt, zusetzt.

Das erfindungsgemäße Verfahren wird bevorzugt bei einer Temperatur im Bereich von -20 bis 400°C, besonders im Bereich von 0 bis 350 °C, weiter besonders im Bereich von 10 bis 250 °C, durchführt.

In weiteren Ausführungsformen liegt die Untergrenze eines bevorzugten Temperaturbereichs bei 20, 50, 100 oder 200 °C, die Obergrenze bei 220 oder 150 °C.

Dieser gefundene Einfluss von Alkoholen auf die Farbentwicklung von N-Alkyl-lactamen ist neu. Im Unterschied zu den technischen Lehren, wie z. B. der thermischen Stabilisierung von N-Methyl-pyrrolidon oberhalb 250 °C aus US 4,168,226 (Exxon Research & Engineering Co.), hat der Alkoholzusatz bzw. die Alkoholfreisetzung gemäß des erfindungsgemäßen Verfahrens keinen messbaren positiven Einfluss auf die Geschwindigkeit der Zersetzung der N-Alkyl-lactame, aber auf deren Farbqualität, z.B. die Farbentwicklung.

Der genau Wirkungsmechanismus der Alkoholzusätze ist noch nicht bekannt. Erfindungsgemäß können alle N-Alkyl-lactame auf diese Weise behandelt werden.
Der Lactamring der N-Alkyl-lactame kann z. B. vier bis acht Kohlenstoffatome aufweisen, bevorzugt vier (Pyrrolidone), fünf (Piperidone) oder sechs (Caprolactame), besonders bevorzugt können 2-Pyrrolidone und 2-Piperidone eingesetzt werden.

Bevorzugt werden im erfindungsgemäßen Verfahren N-Alkyl-lactame der Formel I eingesetzt 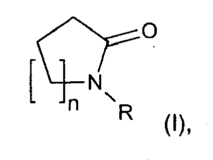 in der R einen
linearen oder verzweigten gesättigten aliphatischen Rest, bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl, besonders bevorzugt C₁₋₈-Alkyl, wie Methyl, Ethyl, n-Propyl, iso-Propyl, n-Butyl, iso-Butyl, sec.-Butyl, tert.-Butyl, n-Pentyl, n-Hexyl, n-Octyl und 2-Ethyl-hexyl, ganz besonders bevorzugt C₁₋₄-Alkyl, wie Methyl, Ethyl, n-Propyl, iso-Propyl, n-Butyl, iso-Butyl, sec.-Butyl, tert.-Butyl,
oder einen
gesättigten cycloaliphatischen Rest mit 3 bis 12 C-Atomen, bevorzugt C₄₋₈-Cycloalkyl, wie Cyclobutyl, Cyclopentyl, Cyclohexyl, Cycloheptyl und Cyclooctyl, besonders bevorzugt Cyclopentyl und Cyclohexyl,
und n eine ganze Zahl von 1 bis 5 bedeutet
und wobei die Kohlenstoffatome des heterocyclischen Rings des N-substituierten Lactams ein bis zwei unter den Bedingungen inerte Substituenten, z. B. Alkylreste, wie z. B. C₁₋₈-Alkylreste, die voneinander unabhängig sind, bevorzugt einen C₁₋₈-Alkylrest, besonders einen C₁₋₄-Alkylrest, tragen können.

Beispiele für C₁₋₈-Alkylreste, die die Kohlenstoffatome des heterocyclischen Rings des N-substituierten Lactams tragen können, sind:
Methyl, Ethyl, n-Propyl, iso-Propyl, n-Butyl, iso-Butyl, sec.-Butyl, tert.-Butyl, n-Pentyl, iso-Pentyl und 2-Ethyl-hexyl,
wie z. B. in 1,5-Dimethyl-2-pyrrolidon und 1-Ethyl-5-methyl-2-pyrrolidon.

Im erfindungsgemäßen Verfahren werden besonders bevorzugt N-Alkyl-lactame der Formel I eingesetzt 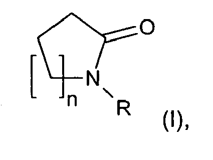 in der R C₁₋₄-Alkyl wie oben beschrieben bedeutet und n 1, 2 oder 3 bedeutet und wobei die Kohlenstoffatome des heterocyclischen Rings des N-substituierten Lactams einen C₁₋₄-Alkylrest, besonders Methyl- oder Ethylrest, tragen können.

Die eingesetzten N-Alkyl-lactame können eine Reinheit von ≥ 90 Gew.-% aufweisen, bevorzugt ≥ 95 Gew.-%, weiter bevorzugt ≥ 99 Gew.-%.
Als mögliche Verunreinigungen der N-Alkyl-lactame sind entsprechende Lactone (wie z. B. gamma-Butyrolacton), entsprechende N-unsubstituierte Lactame (z..B. Pyrrolidon), organische Peroxide, entsprechende Monoalkylamine (wie z. B. Monoethylamin), entsprechende cyclische N-Alkylimide (z. B. N-Alkylsuccinimide), Wasser, die bevorzugt jeweils in Mengen ≤ 1 Gew.-% vorliegen können, möglich und nicht störend.

Als Alkohole, die zur Erhöhung der Farbstabilität zugesetzt bzw. freigesetzt werden, kommen alle Alkohole mit einer oder mehreren Hydroxyfunktion/en in Frage, insbesondere solche mit 1 bis 10, bevorzugt 1 bis 8 Kohlenstoffatomen, weiter bevorzugt 1 bis 3 Kohlenstoffatomen, ganz besonders bevorzugt 1 bis 2 Kohlenstoffatomen.

Bevorzugte monofunktionelle (einwertige) Alkohole sind Methanol, Ethanol, n-Propanol, iso-Propanol, n-Butanol, n-Pentanol, n-Hexanol, n-Heptanol und n-Octanol.

Besonders wirksame polyfunktionelle (mehrwertige) Alkohole sind 1,2-Ethylenglykol, 1,2-Propandiol, 1,3-Propandiol, Glycerin, Pentaerythritol und Sorbitol.

Ganz besonders bevorzugte zugesetzte bzw. freigesetzte Alkohole sind Methanol und 1,2-Ethylenglykol.

Im Sinne der Erfindung können auch solche Stoffe zugesetzt werden, die Alkohole erst nach einer chemischen Umwandlung freisetzen, beispielsweise mit Wasser (Hydrolyse) oder Aminen (Aminolyse), das/die in N-Alkyl-lactamen in Spuren vorhanden sein kann/können oder zugesetzt werden kann/können.

Zu diesen Alkoholquellen, im folgenden auch als Alkoholvorläufer bezeichnet, zählen z. B. Dimethoxymethan, Diethoxymethan, Tetramethoxymethan, Tetraethoxymethan, Trimethylorthoformiat und Triethylorthoformiat, die z.B. durch Hydrolyse Alkohole und Ester oder Alkohole und Formaldehyd freisetzen.

Natürlich kann auch eine Mischung der Alkohole und/oder Alkoholvorläufer eingesetzt werden. Die Reinheit der zugesetzten Alkohole oder Alkoholvorläufer ist dabei nicht kritisch und Verunreinigungen wie z. B. Wasser, Ether, Ester und Kohlenwasserstoffe werden toleriert.

Die Alkoholzugabe bzw. Alkoholvorläuferzugabe kann auf mehrere Arten geschehen, beispielsweise können die Alkohole und/oder Alkoholvorläufer dem N-Alkyl-lactam direkt nach dessen Synthese sowie nach oder bei der Reinigung des Lactams zugesetzt werden. Ebenso ist eine Zugabe bei Abfüllung des Lactams in lager- oder transportfähige Gebinde möglich. Die Zugabe der Alkohole bzw. Alkoholvorläufer kann sowohl diskontinuierlich als auch kontinuierlich erfolgen.

Gegenstand der Erfindung sind gemäß den obigen Ausführungen auch
Mischungen enthaltend mindestens 99,0 Gew.-%, bevorzugt ≥ 99,2 Gew.-%, besonders ≥ 99,5 Gew.-%, eines N-Alkyl-lactams und im Bereich von 100 bis 5000 Gew.-ppm, bevorzugt 200 bis 2000 Gew.-ppm, besonders 300 bis 1000 Gew.-ppm, eines C₁₋₁₀-Alkohols, bevorzugt C₁₋₈-Alkohols, besonders C₁₋₃-Alkohols, ganz besonders C₁₋₂-Alkohols,
oder eines Acetals, Aminals oder eines Orthoesters, das/der im Bereich von 100 bis 5000 Gew.-ppm, bevorzugt 200 bis 2000 Gew.-ppm, besonders 300 bis 1000 Gew.-ppm, eines C₁₋₁₀-Alkohols, bevorzugt C₁₋₈-Alkohols, besonders C₁₋₃-Alkohols, ganz besonders C₁₋₂-Alkohols, freisetzt,

insbesondere Mischungen enthaltend mindestens 99,0 Gew.-%, bevorzugt ≥ 99,2 Gew.-%, besonders ≥ 99,5 Gew.-%, N-Ethyl-2-pyrrolidon (NEP) und im Bereich von 100 bis 5000 Gew.-ppm, bevorzugt 200 bis 2000 Gew.-ppm, besonders 300 bis 1000 Gew.-ppm, Methanol oder 1,2-Ethylenglykol, insbesondere Mischungen enthaltend mindestens 99,0 Gew.-%, bevorzugt ≥ 99,2 Gew.-%, besonders ≥ 99,5 Gew.-%, N-Ethyl-E-caprolactam (NEC) und im Bereich von 100 bis 5000 Gew.-ppm, bevorzugt 200 bis 2000 Gew.-ppm, besonders 300 bis 1000 Gew.-ppm, Methanol oder 1,2-Ethylenglykol,

insbesondere Mischungen enthaltend mindestens 99,0 Gew.-%, bevorzugt ≥ 99,2 Gew.-%, besonders ≥ 99,5 Gew.-%, 1,5-Dimethyl-2-pyrrolidon und im Bereich von 100 bis 5000 Gew.-ppm, bevorzugt 200 bis 2000 Gew.-ppm, besonders 300 bis 1000 Gew.-ppm, Methanol oder 1,2-Ethylenglykol,

insbesondere Mischungen enthaltend mindestens 99,0 Gew.-%, bevorzugt ≥ 99,2 Gew.-%, besonders ≥ 99,5 Gew.-%, 1-Ethyl-5-methyl-2-pyrrolidon und im Bereich von 100 bis 5000 Gew.-ppm, bevorzugt 200 bis 2000 Gew.-ppm, besonders 300 bis 1000 Gew.-ppm, Methanol oder 1,2-Ethylenglykol,

insbesondere Mischungen enthaltend mindestens 99,0 Gew.-%, bevorzugt ≥ 99,2 Gew.-%, besonders ≥ 99,5 Gew.-%, 1-Methyl-2-piperidon und im Bereich von 100 bis 5000 Gew.-ppm, bevorzugt 200 bis 2000 Gew.-ppm, besonders 300 bis 1000 Gew.-ppm, Methanol oder 1,2-Ethylenglykol.

Alle ppm-Angaben beziehen sich auf das Gewicht (Gew.-ppm).

### Beispiele

Die APHA-Farbzahlen (Hazen) wurden bestimmt nach DIN EN ISO 6271.

GC-Methode zur Bestimmung der Reinheit der N-Alkyl-lactame:
Die Lactame wurden unverdünnt in den GC-Chromatographen (Firma HP, Trägergas: Wasserstoff) auf eine 30 m DB5-Säule (Firma J+W) gespritzt und bei Ofentemperaturen von 60 °C bis 260 °C (Aufheizrate 16 Kelvin pro Minute bis 220 °C, dann 20 Kelvin pro Minute bis 260 °C) mit einem Flammenionisationsdetektor (Temperatur: 290 °C) analysiert. Die Reinheit wurde durch Integration der Signale des Chromatogramms bestimmt.

Die Bestimmung des Gesamtgehalts an Alkohol im N-Alkyl-lactam, einschließlich der durch Hydrolyse freisetzbaren Alkohole, wurde durch Headspace-Gaschromatographie durchgeführt. Vor dem Einspritzen in den Gaschromatographen wurden die Proben (ungefähr 100 mg) mit verdünnter, wässriger Phosphorsäure (ungefähr 1 ml) versetzt und 1 h auf 80 °C temperiert, um die Gesamtmenge an Alkohol freizusetzen. Der Dampfraum wurde in einen Gaschromatographen (Firma HP, Trägergas: Wasserstoff) mit einer DB1-Säule (Firma J+W) und einem Flammenionisationsdetektor injiziert. Die Auswertung erfolgte durch Integration der Signale des Chromatogramms. Die Kalibrierung erfolgte durch Zugabe definierter Mengen des zu untersuchenden Alkohols und Analyse nach analoger Vorschrift.

### Beispiel 1

N-Ethyl-pyrrolidon (250 ml, Reinheit 99,69 % laut GC, Farbzahl nach Hazen: 12 APHA) wurde mit Methanol (500 ppm) versetzt und in einem 500 ml-Glaskolben mit aufgesetztem Rückflusskühler und Trockenrohr in Gegenwart von Luft auf 100 °C erhitzt. Nach 72 Stunden (h) hatte die Reinheit abgenommen (auf 97,55 % laut GC), während die Farbzahl auf 208 APHA angestiegen war.

### Beispiel 2

N-Ethyl-pyrrolidon (250 ml, Reinheit 99,69 % laut GC, Farbzahl nach Hazen: 12 APHA) wurde mit 1,2-Ethylenglycol (500 ppm) versetzt und in einem 500 ml-Glaskolben mit aufgesetztem Rückflusskühler und Trockenrohr in Gegenwart von Luft auf 100 °C erhitzt. Nach 72 h hatte die Reinheit abgenommen (auf 97,66 % laut GC), während die Farbzahl auf 176 APHA angestiegen war.

### Vergleichsbeispiel 1

N-Ethyl-pyrrolidon (250 ml, Reinheit 99,69 % laut GC, Farbzahl nach Hazen: 7 APHA) wurde ohne Zusätze in einem 500 ml-Glaskolben mit aufgesetztem Rückflusskühler und Trockenrohr in Gegenwart von Luft auf 100 °C) erhitzt. Nach 72 h hatte die Reinheit abgenommen (auf 97,77 % laut GC), während die Farbzahl auf 284 APHA angestiegen war.

### Beispiel 3

N-Ethyl-pyrrolidon (10 ml, Reinheit 99,59 % laut GC, Farbzahl nach Hazen: 7 APHA) wurde mit Dimethyoxymethan (500 ppm) versetzt. Die Mischung wurde in einem gasdicht verschlossenen 20 ml-Glasautoklav auf 100 °C erhitzt. Nach 72 Stunden (h) hatte die Reinheit abgenommen (auf 99,37 % laut GC), während die Farbzahl auf 22 APHA angestiegen war.

### Vergleichsbeispiel 2

N-Ethyl-pyrrolidon (10 ml, Reinheit 99,59 % laut GC, Farbzahl nach Hazen: 7 APHA) wurde ohne Zusätze in einem gasdicht verschlossenen 20 ml-Glasautoklav auf 100 °C erhitzt. Nach 72 Stunden (h) hatte die Reinheit abgenommen (auf 99,27 % laut GC), während die Farbzahl auf 32 APHA angestiegen war.

### Beispiel 4

N-Methyl-piperidon (10 ml, Reinheit 99,20 % laut GC, Farbzahl nach Hazen: 18 APHA) wurde mit Methanol (500 ppm) versetzt. Die Mischung wurde in einem gasdicht verschlossenen 20 ml-Glasautoklav auf 100 °C erhitzt. Nach 72 Stunden (h) hatte die Reinheit abgenommen (auf 98,82 % laut GC), während die Farbzahl auf 172 APHA angestiegen war.

### Vergleichsbeispiel 3

N-Methyl-piperidon (10 ml, Reinheit 99,20 % laut GC, Farbzahl nach Hazen: 18 APHA) wurde ohne Zusätze in einem gasdicht verschlossenen 20 ml-Glasautoklav auf 100 °C erhitzt. Nach 72 Stunden (h) hatte die Reinheit abgenommen (auf 98,73 % laut GC), während die Farbzahl auf 197 APHA angestiegen war.

### Beispiel 5

1,5-Dimethyl-2-pyrrolidon (10 ml, Reinheit 99,72 % laut GC, Farbzahl nach Hazen: 5 APHA) wurde mit Methanol (500 ppm) versetzt. Die Mischung wurde in einem gasdicht verschlossenen 20 ml-Glasautoklav auf 100 °C erhitzt. Nach 72 Stunden (h) hatte die Reinheit abgenommen (auf 99,53 % laut GC), während die Farbzahl auf 404 APHA angestiegen war.

### Vergleichsbeispiel 4

1 ,5-Dimethyl-2-pyrrolidon (10 ml, Reinheit 99,72 % laut GC, Farbzahl nach Hazen: 5 APHA) wurde ohne Zusätze in einem gasdicht verschlossenen 20 ml-Glasautoklav auf 100 °C erhitzt. Nach 72 Stunden (h) hatte die Reinheit abgenommen (auf 99,46 % laut GC), während die Farbzahl auf 669 APHA angestiegen war.

## Patentansprüche

1. Verfahren zur Herstellung eines N-Alkyl-lactams, **dadurch gekennzeichnet, dass** man dem N-Alkyl-lactam 0,01 bis 10 Gew.-% eines C₁₋₁₀-Alkohols oder eine Verbindung, die 0,01 bis 10 Gew.-% eines C₁₋₁₀-Alkohols freisetzt, zusetzt.

2. Verfahren nach Anspruch 1 zur Herstellung eines N-Alkyl-2-pyrrolidons oder N-Alkyl-2-piperidons.

3. Verfahren nach Anspruch 1 zur Herstellung eines N-(C₁₋₈-Alkyl)-2-pyrrolidons oder N-(C₁₋₈-Alkyl)-2-piperidons.

4. Verfahren nach Anspruch 1 zur Herstellung von N-Ethyl-2-pyrrolidon (NEP), 1,5-Dimethyl-2-pyrrolidon, 1-Ethyl-5-methyl-2-pyrrolidon, 1-Methyl-2-piperidon oder N-Ethyl-ε-caprolactam (NEC).

5. Verfahren nach einem der vorhergehenden Ansprüche, **dadurch gekennzeichnet, dass** es sich bei dem C₁₋₁₀-Alkohol um Methanol, Ethanol, n-Propanol, iso-Propanol, n-Butanol, n-Pentanol, n-Hexanol, n-Heptanol, n-Octanol, 1,2-Ethylenglykol, 1,2-Propandiol, 1,3-Propandiol, Glycerin, Pentaerythritol und/oder Sorbitol handelt.

6. Verfahren nach einem der vorhergehenden Ansprüche, **dadurch gekennzeichnet, dass** es sich bei der Verbindung, die den C₁₋₁₀-Alkohol freisetzt, um ein Acetal, Aminal oder einen Orthoester handelt.

7. Verfahren nach dem vorhergehenden Anspruch, **dadurch gekennzeichnet, dass** es sich bei der Verbindung, die den C₁₋₁₀-Alkohol freisetzt, um Dimethoxymethan, Diethoxymethan, Tetramethoxymethan, Tetraethoxymethan, Trimethylorthoformiat und/oder Triethylorthoformiat handelt.

8. Verfahren nach einem der vorhergehenden Ansprüche, **dadurch gekennzeichnet, dass** man dem N-Alkyl-lactam 0,02 bis 2 Gew.-% eines C₁₋₁₀-Alkohols oder eine Verbindung, die 0,02 bis 2 Gew.-% eines C₁₋₁₀-Alkohols freisetzt, zusetzt.

9. Verfahren nach einem der Ansprüche 1 bis 7, **dadurch gekennzeichnet, dass** man dem N-Alkyl-lactam 0,03 bis 1 Gew.-% eines C₁₋₁₀-Alkohols oder eine Verbindung, die 0,03 bis 1 Gew.-% eines C₁₋₁₀-Alkohols freisetzt, zusetzt.

10. Verfahren nach einem der vorhergehenden Ansprüche, **dadurch gekennzeichnet, dass** man es bei einer Temperatur im Bereich von -20 bis 400 °C durchführt.

11. Verfahren nach einem der Ansprüche 1 bis 9, **dadurch gekennzeichnet, dass** man es bei einer Temperatur im Bereich von 10 bis 250 °C durchführt.

12. Mischung enthaltend mindestens 99,0 Gew.-% eines N-Alkyl-lactams und im Bereich von 100 bis 5000 Gew.-ppm eines C₁₋₁₀-Alkohols oder eines Acetals, Aminals oder eines Orthoesters, das/der im Bereich von 100 bis 5000 Gew.-ppm eines C₁₋₁₀-Alkohols freisetzt.

13. Mischung enthaltend mindestens 99,0 Gew.-% N-Ethyl-2-pyrrolidon (NEP) und im Bereich von 100 bis 5000 Gew.-ppm Methanol oder 1,2-Ethylenglykol.

14. Mischung enthaltend mindestens 99,0 Gew.-% N-Ethyl-ε-caprolactam (NEC) und im Bereich von 100 bis 5000 Gew.-ppm Methanol oder 1,2-Ethylenglykol.

15. Mischung enthaltend mindestens 99,0 Gew.-% 1,5-Dimethyl-2-pyrrolidon und im Bereich von 100 bis 5000 Gew.-ppm Methanol oder 1,2-Ethylenglykol.

16. Mischung enthaltend mindestens 99,0 Gew.-% 1-Ethyl-5-methyl-2-pyrrolidon und im Bereich von 100 bis 5000 Gew.-ppm Methanol oder 1,2-Ethylenglykol.

17. Mischung enthaltend mindestens 99,0 Gew.-% 1-Methyl-2-piperidon und im Bereich von 100 bis 5000 Gew.-ppm Methanol oder 1,2-Ethylenglykol.

## Claims

1. A process for preparing an N-alkyllactam, which comprises adding to the N-alkyllactam from 0.01 to 10% by weight of a C₁₋₁₀-alcohol or a compound which releases from 0.01 to 10% by weight of a C₁₋₁₀-alcohol.

2. The process according to claim 1 for preparing an N-alkyl-2-pyrrolidone or N-alkyl-2-piperidone.

3. The process according to claim 1 for preparing an N-(C₁_₈-alkyl)-2-pyrrolidone or N-(C₁₋₈-alkyl)-2-piperidone.

4. The process according to claim 1 for preparing N-ethyl-2-pyrrolidone (NEP), 1,5-dimethyl-2-pyrrolidone, 1-ethyl-5-methyl-2-pyrrolidone, 1-methyl-2-piperidone or N-ethyl-ε-caprolactam (NEC).

5. The process according to any of the preceding claims, wherein the C₁₋₁₀-alcohol is methanol, ethanol, n-propanol, isopropanol, n-butanol, n-pentanol, n-hexanol, n-heptanol, n-octanol, 1,2-ethylene glycol, 1,2-propanediol, 1,3-propanediol, glycerol, pentaerythritol and/or sorbitol.

6. The process according to any of the preceding claims, wherein the compound which releases the C₁₋₁₀-alcohol is an acetal, aminal or an orthoester.

7. The process according to the preceding claim, wherein the compound which releases the C₁₋₁₀-alcohol is dimethoxymethane, diethoxymethane, tetramethoxymethane, tetraethoxymethane, trimethyl orthoformate and/or triethyl orthoformate.

8. The process according to any of the preceding claims, wherein from 0.02 to 2% by weight of a C₁₋₁₀-alcohol or a compound which releases from 0.02 to 2% by weight of a C₁₋₁₀-alcohol is added to the N-alkyllactam.

9. The process according to any of claims 1 to 7, wherein from 0.03 to 1% by weight of a C₁₋₁₀-alcohol or a compound which releases from 0.03 to 1% by weight of a C₁₋₁₀-alcohol is added to the N-alkyllactam.

10. The process according to any of the preceding claims, which is performed at a temperature in the range from -20 to 400°C.

11. The process according to any of claims 1 to 9, which is performed at a temperature in the range from 10 to 250°C.

12. A mixture comprising at least 99.0% by weight of an N-alkyllactam and in the range from 100 to 5000 ppm by weight of a C₁₋₁₀-alcohol or of an acetal, aminal or of an orthoester which releases in the range from 100 to 5000 ppm by weight of a C₁₋₁₀-alcohol.

13. A mixture comprising at least 99.0% by weight of N-ethyl-2-pyrrolidone (NEP) and in the range from 100 to 5000 ppm by weight of methanol or 1,2-ethylene glycol.

14. A mixture comprising at least 99.0% by weight of N-ethyl-2-caprolactam (NEC) and in the range from 100 to 5000 ppm by weight of methanol or 1,2-ethylene glycol.

15. A mixture comprising at least 99.0% by weight of 1,5-dimethyl-2-pyrrolidone and in the range from 100 to 5000 ppm by weight of methanol or 1,2-ethylene glycol.

16. A mixture comprising at least 99.0% by weight of 1-ethyl-5-methyl-2-pyrrolidone and in the range from 100 to 5000 ppm by weight of methanol or 1,2-ethylene glycol.

17. A mixture comprising at least 99.0% by weight of 1-methyl-2-piperidone and in the range from 100 to 5000 ppm by weight of methanol or 1,2-ethylene glycol.

## Revendications

1. Procédé pour la préparation d'un N-alkyl-lactame, **caractérisé en ce qu'**on ajoute au N-alkyl-lactame 0,01 à 10 % en poids d'un alcool en C₁-C₁₀ ou d'un composé qui libère 0,01 à 10 % en poids d'un alcool en C₁-C₁₀.

2. Procédé selon la revendication 1, pour la préparation d'une N-alkyl-2-pyrrolidone ou N-alkyl-2-pipéridone.

3. Procédé selon la revendication 1, pour la préparation d'une N-[alkyl(C₁-C₈)]-2-pyrrolidone ou N-[alkyl(C₁-C₈)]-2-pipéridone.

4. Procédé selon la revendication 1, pour la préparation de N-éthyl-2-pyrrolidone (NEP), 1,5-diméthyl-2-pyrrolidone, 1-éthyl-5-méthyl-2-pyrrolidone, 1-méthyl-2-pipéridone ou N-éthyl-ε-caprolactame (NEC).

5. Procédé selon l'une quelconque des revendications précédentes, **caractérisé en ce que** l'alcool en C₁-C₁₀ consiste en le méthanol, l'éthanol, le n-propanol, l'isopropanol, le n-butanol, le n-pentanol, le n-hexanol, le n-heptanol, le n-octanol, le 1,2-éthylèneglycol, le 1,2-propanediol, le 1,3-propanediol, le glycérol, le pentaérythritol et/ou le sorbitol.

6. Procédé selon l'une quelconque des revendications précédentes, **caractérisé en ce que** le composé qui libère l'alcool en C₁-C₁₀ est un acétal, un aminal ou un orthoester.

7. Procédé selon la revendication précédente, **caractérisé en ce que** le composé qui libère l'alcool en C₁-C₁₀ consiste en le diméthoxyméthane, le diéthoxyméthane, le tétraméthoxyméthane, le tétraéthoxyméthane, l'orthoformiate de triméthyle et/ou l'orthoformiate de triéthyle.

8. Procédé selon l'une quelconque des revendications précédentes, **caractérisé en ce qu'**on ajoute au N-alkyl-lactame 0,02 à 2 % en poids d'un alcool en C₁-C₁₀ ou d'un composé qui libère 0,02 à 2 % en poids d'un alcool en C₁-C₁₀.

9. Procédé selon l'une quelconque des revendications 1 à 7, **caractérisé en ce qu'**on ajoute au N-alkyl-lactame 0,03 à 1 % en poids d'un alcool en C₁-C₁₀ ou d'un composé qui libère 0,03 à 1 % en poids d'un alcool en C₁-C₁₀.

10. Procédé selon l'une quelconque des revendications précédentes, **caractérisé en ce qu'**on l'effectue à une température dans la plage de -20 à 400 °C.

11. Procédé selon l'une quelconque des revendications 1 à 9, **caractérisé en ce qu'**on l'effectue à une température dans la plage de 10 à 250 °C.

12. Mélange contenant au moins 99,0 % en poids d'un N-alkyl-lactame et dans la plage de 100 à 5 000 ppm en poids d'un alcool en C₁-C₁₀ ou d'un acétal, d'un aminal ou d'un orthoester, qui libère dans la plage de 100 à 5 000 ppm en poids d'un alcool en C₁-C₁₀.

13. Mélange contenant au moins 99,0 % en poids de N-éthyl-2-pyrrolidone (NEP) et dans la plage de 100 à 5 000 ppm en poids de méthanol ou de 1,2-éthylèneglycol.

14. Mélange contenant au moins 99,0 % en poids de N-éthyl-ε-caprolactame (NEC) et dans la plage de 100 à 5 000 ppm en poids de méthanol ou de 1,2-éthylèneglycol.

15. Mélange contenant au moins 99,0 % en poids de 1,5-diméthyl-2-pyrrolidone et dans la plage de 100 à 5 000 ppm en poids de méthanol ou de 1,2-éthylèneglycol.

16. Mélange contenant au moins 99,0 % en poids de 1-éthyl-5-méthyl-2-pyrrolidone et dans la plage de 100 à 5 000 ppm en poids de méthanol ou de 1,2-éthylèneglycol.

17. Mélange contenant au moins 99,0 % en poids de 1-méthyl-2-pipéridone et dans la plage de 100 à 5 000 ppm en poids de méthanol ou de 1,2-éthylèneglycol.
